# DEMANDE DE BREVET EUROPEEN

(11) **EP 1 350 491 A1**
(43) Date de publication de la demande: **08.10.2003**
(21) Numéro de dépôt: 03290764.4
(22) Date de dépôt: 26.03.2003
(51) Int. Cl.: A61F 9/007, A61B 19/02

(54) **Dispositif de chirurgie ophtalmique**

(30) Priorité: 26.03.2002 FR 0203782
(71) Demandeur: Promepla S.A., 98000 Monaco (MC)
(72) Inventeur: Brocq, Alain, 78112 Fourqueux (FR); Danchin, Marc, 06190 Roquebrune Cap Martin (FR)
(74) Mandataire: Wagret, Frédéric

(57) **Abrégé**

La présente invention concerne un dispositif de chirurgie ophtalmique comportant un manche (1) présentant à l'une de ses extrémités une première partie active (2) et à l'autre de ses extrémités une seconde partie active (3), caractérisé en ce que des moyens de protection amovibles (4, 5) sont associés à au moins l'une des parties actives (2, 3).

Les zones fonctionnelles du dispositif ainsi protégées ne donc risquent pas d'être altérées avant utilisation.

## Description

La présente invention concerne un dispositif de chirurgie ophtalmique, plus connu sous l'appellation de « pic à corps étranger ».

Ce type de dispositif est un outil de précision comportant un manche et deux parties actives, situées respectivement à chaque extrémité dudit manche.

Ces parties actives peuvent comprendre, par exemple, une pointe à l'une des extrémités et une sorte de lame du type scalpel à l'autre extrémité.

Selon l'art antérieur, les pics à corps étranger sont réalisés en métal, tel que de l'acier, afin d'assurer notamment une grande précision dans les détails des formes des parties actives.

Ce type de dispositif connu est relativement onéreux et nécessite un grand nombre de traitements pré et post opératoires, tels qu'un nettoyage, des vérifications diverses ainsi que la stérilisation.

De plus, les différentes manipulations de traitement, ainsi que lors du transport et du conditionnement, entraînent une certaine usure des parties actives, ainsi que des risques de corrosion.

Par ailleurs, les différentes manipulations ainsi qu'un traitement mal effectué peuvent entraîner des risques de contamination.

Dans ce contexte, la présente invention pallie les inconvénients de l'art antérieur en proposant un dispositif de chirurgie ophtalmique peu onéreux et où les parties actives ne risquent pas d'être altérées.

A cette fin, selon l'invention, le dispositif de chirurgie ophtalmique comportant un manche présentant à l'une de ses extrémités une première partie active et à l'autre de ses extrémités une seconde partie active est caractérisé en ce que des moyens de protection amovibles sont associés à au moins l'une des parties actives.

Selon une forme avantageuse de réalisation, les moyens de protection amovibles comportent des éléments sécables comprenant des languettes présentant un évidement de manière à recevoir la partie active correspondante.

De préférence, les éléments sécables sont associés à la partie active correspondante par une ou plusieurs liaisons ponctuelles aptes à être brisées et comportent une zone de préhension.

Selon une forme particulière de réalisation, la première partie active est conformée en pointe tandis que la seconde partie active est conformée sensiblement en lame.

Avantageusement, le dispositif est réalisé en un matériau thermoplastique, tel que du polycarbonate.

L'invention sera mieux comprise à la lumière de la description qui suit, se rapportant à un exemple de réalisation illustratif et en aucun cas limitatif, en référence aux dessins annexés dans lesquels :
- la Figure 1 est une vue générale d'une forme de réalisation du dispositif de chirurgie ophtalmique selon l'invention ;
- la Figure 2 est une vue de détail d'une des parties actives du dispositif selon l'invention ;
- la Figure 3 est une vue en détail de l'autre partie active du dispositif selon l'invention.

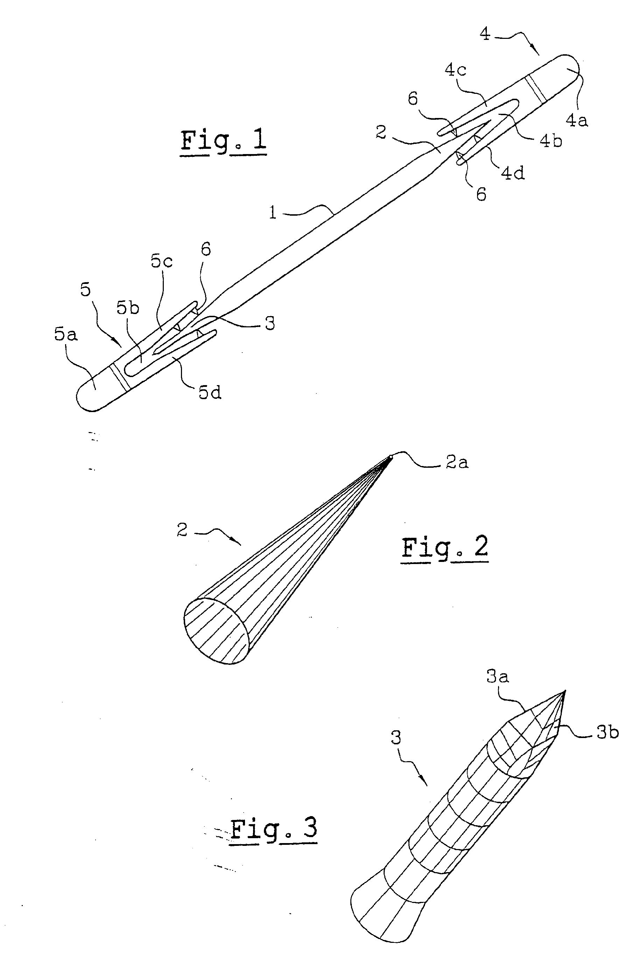

La figure 1 est une représentation d'une forme de réalisation du dispositif de chirurgie ophtalmique selon l'invention.

Ledit dispositif, de forme sensiblement longiligne comporte un manche 1 sensiblement cylindrique dont chacune des extrémités est terminée par une partie active (2, 3), destinée à réaliser les opérations de chirurgie.

Lesdites parties actives (2, 3) peuvent prendre différentes formes, et seront décrites en détail ultérieurement.

Afin de protéger lesdites parties actives (2, 3), ces dernières sont associées respectivement à des moyens de protection amovibles (4, 5).

Il est entendu que, même si la forme de réalisation illustrée sur la figure 1 montre des moyens de protection amovibles (4, 5) associés à chacune des parties actives (2, 3), seule l'une desdites parties actives (2, 3) peut être associée à des moyens de protection amovibles (4, 5).

Lesdits moyens de protection amovibles (4, 5) présentent chacun la forme d'une languette sécable apte à être désolidarisée aisément du corps du dispositif.

Les languettes sécables (4, 5) présentent à leur extrémité distale, extrémité opposée à celle associée à la partie active correspondante, une zone (4a, 5a) sensiblement plane, destinée à être saisie par les doigts de l'utilisateur, en vue de la désolidarisation des languettes sécables (4, 5) du corps du dispositif.

L'extrémité proximale des languettes sécables (4, 5) est sensiblement conformée en V, afin de ménager une ouverture respectivement (4b 5 b), de forme sensiblement complémentaire aux parties actives correspondantes (2 et 3).

Chacune desdites parties actives (2, 3) est ainsi protégée par les languettes sécables (4, 5), en étant insérée respectivement dans les ouvertures (4b, 5b).

Les ouvertures (4b, 5b), conformées sensiblement en V, forment ainsi chacune une paire de pattes (4c, 4d) et (5c, 5d).

Chacune des pattes (4c, 4d, 5c, 5d) est liée à la partie active (2, 3) correspondante par l'intermédiaire d'une ou plusieurs liaisons ponctuelles 6 de dimensions suffisamment faibles pour pouvoir être aisément brisées.

Ainsi, sur la forme de réalisation du dispositif illustré à la Figure 1, la patte 4c est associée à la partie active 2 par l'intermédiaire d'une liaison ponctuelle 6, tandis que la patte 4d est associée à la même partie active 2 par l'intermédiaire de deux liaisons ponctuelles 6.

De même, la patte 5c est associée à la partie active 3 par l'intermédiaire de deux liaisons ponctuelles 6, tandis que la patte 5d est associée à la partie active 3 par l'intermédiaire d'une unique liaison ponctuelle 6.

Il est entendu que les dimensions des liaisons ponctuelles 6, permettant d'associer de manière amovible les languettes sécables (4, 5) aux parties actives correspondantes (2, 3), peuvent varier.

De même, lesdites languettes sécables (4, 5) peuvent être associées aux parties actives correspondantes (2, 3) par l'intermédiaire d'une liaison linéaire apte à être cependant aisément brisée en vue de l'utilisation de la partie active correspondante.

La figure 2 est une représentation détaillée et à grande échelle d'une forme particulière de réalisation de la partie active 2.

Ladite partie active 2 présente une forme sensiblement conique, afin de définir une pointe dont l'extrémité est très fine.

La figure 3 est une représentation à grande échelle et détaillée de la partie active 3, présentant une forme sensiblement cylindrique, dont l'extrémité 3a possède une taille particulière de manière à définir des facettes et notamment une facette 3b sensiblement biseautée.
Ainsi conformée, la partie active 3 peut être utilisée comme une lame de précision.

En référence à nouveau à la figure 1, le manche 1 du dispositif selon l'invention présente avantageusement une surface légèrement granuleuse, afin de faciliter sa préhension et sa manipulation.

Selon une forme de réalisation particulièrement avantageuse, le dispositif de chirurgie ophtalmique selon l'invention est réalisé en une matière thermoplastique, permettant d'obtenir en une pièce unique l'ensemble du dispositif comprenant le manche 1, avec des parties actives (2, 3) à chacune des extrémités, ainsi que les moyens de protection amovibles (4, 5) correspondants.

Le matériau thermoplastique utilisé peut, par exemple, être du polycarbonate.

Des dimensions possibles du dispositif sont, par exemple, environ 6 mm pour le diamètre du manche 1, 130 mm pour la longueur du dispositif entre les deux extrémités des parties actives (2, 3) et 175 mm pour la longueur totale du dispositif équipé des languettes sécables (4, 5).

Avant toute utilisation, le dispositif selon l'invention est conditionné de manière à conserver son état stérile.

En cas de besoin, le dispositif est sorti de sa pochette de protection et la languette sécable associée à la partie active devant être utilisée est brisée.

Pour ce faire, l'utilisateur maintient simplement le dispositif par le manche 1 et exerce un effort sur la languette sécable, par exemple par l'intermédiaire des zones de préhension (4a, 5a).

Une fois la languette sécable (4, 5) désolidarisée de la partie active correspondante (2, 3), ladite partie active peut être utilisée et ce, sans avoir recours à une stérilisation préalable.

Ainsi, les zones fonctionnelles du dispositif selon l'invention devant entrer en contact avec la partie à opérer sont protégées et ne risquent pas de subir une altération lors de la manipulation ou du conditionnement.

De plus, la réalisation du dispositif selon l'invention en un matériau thermoplastique, tel que le polycarbonate, permet d'envisager un usage unique du dispositif grâce aux importantes économies de fabrication ainsi réalisées.

En conséquence, les traitements pré et post opératoires du dispositif sont supprimés, tout comme les risques de contamination dus à un défaut de traitement, telle qu'une mauvaise stérilisation.

## Revendications

1. Dispositif de chirurgie ophtalmique comportant un manche (1) présentant à l'une de ses extrémités une première partie active (2) et à l'autre de ses extrémités une seconde partie active (3), **caractérisé en ce que** des moyens de protection amovibles (4, 5) sont associés à au moins l'une des parties actives (2, 3).

2. Dispositif selon la revendication 1, **caractérisé en ce que** les deux parties actives (2, 3) sont associées à des moyens de protection amovibles (4, 5).

3. Dispositif selon l'une des revendications 1 ou 2, **caractérisé en ce que** les moyens de protection amovibles (4, 5) comportent des éléments sécables.

4. Dispositif selon la revendication 3, **caractérisé en ce que** les éléments sécables (4, 5) comportent des languettes présentant un évidement (4b, 5b) de manière à recevoir la partie active (2, 3) correspondante.

5. Dispositif selon l'une des revendications 3 ou 4, **caractérisé en ce que** les éléments sécables (4, 5) sont associés à la partie active (2, 3) correspondante par une ou plusieurs liaisons ponctuelles (6) aptes à être brisées.

6. Dispositif selon l'une des revendications 3 à 5, **caractérisé en ce que** les éléments sécables comportent une zone de préhension.

7. Dispositif selon l'une des revendications 1 à 6, **caractérisé en ce que** la première partie active (2) est conformée en pointe.

8. Dispositif selon l'une des revendications 1 à 7, **caractérisé en ce que** la seconde partie active (3) est conformée sensiblement en lame.

9. Dispositif selon l'une des revendications 1 à 8, **caractérisé en ce qu'**il est réalisé en un matériau thermoplastique.

10. Dispositif selon la revendication 9, **caractérisé en ce que** le matériau thermoplastique est du polycarbonate.
